# FASCICULE DE BREVET EUROPEEN

(11) **EP 3 426 354 B1**
(45) Date de publication et mention de la délivrance du brevet: **15.02.2023**
(21) Numéro de dépôt: 17708534.7
(22) Date de dépôt: 07.03.2017
(51) Int. Cl.: A61Q 3/02, A61K 8/25, A61K 8/29, A61K 8/37, A61K 8/81, A61K 8/86

(54) **COMPOSITIONS POUR VERNIS A ONGLES**
ZUSAMMENSETZUNGEN FÜR NAGELLACK
COMPOSITIONS FOR NAIL VARNISH

(30) Priorité: 08.03.2016 FR 1651939
(43) Date de publication de la demande: 16.01.2019
(73) Titulaire: Fiabila SAS, 28130 Maintenon (FR)
(72) Inventeur: MARTINEZ, Francisco, 28000 Chartres (FR)
(74) Mandataire: Hirsch & Associés
(86) Numéro de dépôt international: PCT/EP2017/055262
(87) Numéro de publication internationale: WO 2017/153373

(56) Documents cités:
- EP-A1- 1 608 322
- WO-A2-2012/123123
- DE-A1-102013 102 234
- FR-A1- 2 675 995
- FR-A1- 2 819 176
- US-A- 4 283 324
- US-A1- 2004 022 749
- US-B2- 7 645 444
- DATABASE GNPD [Online] MINTEL; décembre 2003 (2003-12), "Crystal Nail Lacquer", XP002762343, Database accession no. 240854
- DATABASE GNPD [Online] MINTEL; mai 2007 (2007-05), "Nail Varnish", XP002762344, Database accession no. 701193
- DATABASE GNPD [Online] MINTEL; août 2002 (2002-08), "Wet Look Nail Color", XP002762345, Database accession no. 10115251
- DATABASE GNPD [Online] MINTEL; mai 2002 (2002-05), "Water Shine Nail Varnish", XP002762346, Database accession no. 150544
- DATABASE GNPD [Online] MINTEL; mars 2002 (2002-03), "Wet Shine Diamonds Nail Color", XP002762347, Database accession no. 10104834

## Description

### DOMAINE DE L'INVENTION

La présente invention concerne de nouvelles compositions cosmétiques à propriétés améliorées qui sont plus particulièrement utilisables comme, ou pour la préparation de, vernis à ongles, lesdites compositions étant notamment anhydres et exemptes de toute nitrocellulose.

### ARRIERE-PLAN TECHNIQUE

Les vernis à ongles ont peu changé depuis leur conception initiale. Ils peuvent être utilisés non seulement pour habiller et embellir les ongles (maquillage) mais également pour les protéger (soin). Dans le domaine considéré, les ongles s'entendent évidemment tant des ongles des mains que des ongles des pieds.

Pour la formulation d'un vernis à ongle classique, on fait appel à plusieurs ingrédients conventionnels, parmi lesquels figurent :
- un agent filmogène principal cet agent apporte un séchage rapide, l'adhésion sur l'ongle, de la dureté et de la brillance. Il doit toutefois être plastifié car il est cassant.
- une ou plusieurs résines secondaires (polymères) destinées à améliorer la qualité du film : l'agent filmogène principal est complété dans les formulations commerciales par une ou des résines texturantes permettant d'augmenter l'extrait sec et donc de contrôler la quantité de film formé à la surface de l'ongle, et ainsi augmenter certaines propriétés comme le brillant ou l'adhérence. Pendant longtemps, les résines secondaires utilisées étaient des produits obtenus par condensation du toluène sulfonamide et du formaldéhyde, et connus notamment sous le nom commercial de Santolite^{®}. Ces derniers produits, pour des raisons de sécurité (taux de formol résiduel élevé), sont toutefois de plus en plus abandonnés pour être remplacés par d'autres types de résines, comme par exemple des résines polyester, considérées comme plus acceptables d'un point de vue cosmétique.

- un ou plusieurs plastifiants de l'agent filmogène principal pour optimiser et modifier la flexibilité et la souplesse du film.
- un ou plusieurs solvants usuels : ces solvants, de nature le plus souvent organique (acétone, acétate d'éthyle ou acétate de butyle notamment) sont mélangés aux autres ingrédients pour former une composition plus ou moins fluide facilement applicable sur l'ongle avec un pinceau. En particulier, ils doivent être capables de dissoudre l'agent filmogène et les résines secondaires. Ils permettent également d'ajuster la viscosité du vernis à une valeur adaptée. Enfin, la vitesse d'évaporation du solvant (solvant volatile) ne doit être ni trop rapide ni trop lente pour obtenir le meilleur compromis brillance/séchage.
- une ou plusieurs matières colorantes : les colorants solubles dans le support de formulation ne sont pratiquement jamais utilisés dans le domaine des vernis à ongles. Par contre, on fait appel de façon très fréquente voire systématique à des pigments (insolubles dans le milieu de formulation), organiques et/ou minéraux, autorisés par la réglementation dans des applications cosmétiques. Il est connu que les pigments minéraux ont tendance à favoriser le phénomène de sédimentation. Les pigments organiques, quant à eux, sont plus sensibles aux phénomènes de stabilité chimique, dont il sera reparlé un peu plus loin. La composition peut aussi contenir, comme ou en complément des pigments, des nacres et/ou paillettes de différentes natures.

Les compositions de vernis à ongles préparées à partir des ingrédients ci-dessus sont, pour la vente, conditionnées dans des récipients ou flacons, généralement fermés de façon hermétique au moyen de bouchons intégrant des pinceaux destinés à l'application du produit sur l'ongle.

Cette présentation et cette utilisation impliquent deux contraintes particulières aux formulateurs.

La première difficulté est qu'il convient de limiter au maximum, en situation de stockage et de repos du produit, la sédimentation potentielle des pigments et/ou nacres au sein de la composition afin de maintenir son homogénéité dans le flacon. Pour aider à lutter contre ces phénomènes de sédimentation et de stabilité physique, on fait souvent appel à des colloïdes stabilisants. Ces colloïdes sont des argiles organophiles (argiles modifiées), obtenues par substitution des cations minéraux par des cations organiques à partir de qualité d'argiles courantes, telles que bentonite ou montmorillonite.

La deuxième difficulté réside dans le fait que, après son prélèvement dans le flacon par un utilisateur, le vernis à ongle doit pouvoir s'étaler facilement sur l'ongle mais ne plus couler après application, ce qui implique que le vernis puisse retrouver sa viscosité initiale dès l'instant où il n'est plus mis en mouvement avec le pinceau.

De nos jours, la nitrocellulose (encore appelée communément «nitrate de cellulose») est de loin l'agent filmogène principal le plus utilisé dans les vernis à ongles. Cette substance présente en effet de nombreuses propriétés compatibles avec l'application qui en est faite : la transparence, l'adhérence et le séchage, faisant ainsi d'elle une matière première polyvalente, incontournable et presque universelle dans le monde des vernis à ongles commerciaux.

Toutefois, l'emploi de la nitrocellulose n'est pas exempt d'inconvénients. Tout d'abord, il s'agit d'une matière première délicate, voire dangereuse, à fabriquer du fait de son caractère instable (inflammabilité, explosivité). En outre, elle est préparée à partir de cellulose de coton, par attaque directe de balles de coton par un mélange sulfo-nitrique concentré conduisant à l'hydrolyse ménagée du polymère et à l'intégration d'azote dans des proportions déterminées. En raison de son procédé de fabrication, la nitrocellulose contient donc des quantités résiduelles d'acides (acide nitrique, acide sulfurique) qui peuvent poser des problèmes de stabilité lorsque la nitrocellulose est mise en contact avec certains types de pigments, en particulier des pigments organiques (dégradations des teintes).

Par ailleurs, les vernis à ongles nitrocellulosiques ont tendance à jaunir au cours du temps en raison d'une dégradation de la nitrocellulose, entrainant par là des modifications indésirables sur la couleur initiale du vernis à ongle.

Pour ces différentes raisons, de nombreuses tentatives ont été faites pour essayer de substituer cet agent filmogène par d'autres systèmes, même si, aujourd'hui encore, la nitrocellulose, comme rappelé précédemment, continue d'être la base de formulation quasi-universelle des vernis à ongles commerciaux.

Le document US 4 283 324 décrit une composition pour vernis à ongles comprenant du polyvinyl butyral à titre d'agent filmogène principal, une résine complémentaire et un solvant organique, cette composition pouvant être additivée avec des agents colorants et de la bentonite.

Dans le document US 4 409 203, on a proposé de substituer la nitrocellulose par un homopolymère filmogène de type méthacrylate d'éthyle à bas poids moléculaire. Les vernis décrits dans ce document s'avèrent toutefois présenter au final une adhésion insuffisante sur l'ongle, et les films ainsi formés une longévité également insuffisante.

Dans le document FR 2 617 043, on a tenté de substituer la nitrocellulose par un copolymère filmogène résultant de la copolymérisation entre des (méth)acrylates d'alkyle et des (méth)acrylates d'hydroxyalkyle. Toutefois, il s'avère qu'avec ce type d'agent filmogène, on observe un ramollissement du film sur l'ongle dès 24 heures après l'application, ce qui est désavantageux en termes de durabilité et d'acceptabilité pour la consommatrice.

Dans le document EP 2 248 514, on a proposé de remplacer la nitrocellulose par un système filmogène constitué d'un copolymère styrèneanhydride d'acide maléique, éventuellement estérifié, en combinaison avec une résine de type époxy. Toutefois, l'emploi d'un tel système conduit à des produits présentant une sensibilité à l'eau importante du fait des fonctions acides provenant de l'anhydride maléique, ce qui se traduit par une faible durée de vie sur l'ongle.

Le document EP 1 608 322 décrit des vernis à ongles contenant des fibres organiques, comme des fibres d'aramide (« micropulp »). On y recommande d'utiliser, à titre d'agent filmogène principal (« primary resin »), de la nitrocellulose, et tous les exemples ont été réalisés en mettant en oeuvre des quantités importantes de nitrocellulose. Dans les exemples 1 et 2, on décrit des vernis à ongles qui, à côté de la nitrocellulose, contiennent également du polyvinyl butyral en mélange avec de la silice hydratée.

Enfin, dans le document FR 2 675 995, on propose des vernis à ongles, incolores ou colorés, dont l'originalité est de contenir, en plus des ingrédients usuels (solvant, filmogène, résine, plastifiant, etc...) des fibres d'aramide. Les agents filmogènes les plus particulièrement préférés sont les nitrocelluloses et dans les exemples 1 et 3 du document, on décrit des vernis à ongles qui, à côté de quantités importantes de nitrocellulose (plus de 15% en poids), contiennent également du polyvinyl butyral en mélange avec de la silice hydratée.

La présente invention vise à résoudre, pour partie ou en totalité, les problèmes des compositions de vernis à ongles de l'art antérieur, en particulier celles exemptes de nitrocellulose.

Plus précisément, l'invention vise à proposer des compositions pour vernis à ongles, exemptes ou substantiellement exemptes de nitrocellulose, qui soient stables physiquement et chimiquement, en particulier au niveau de leur couleur, et qui présentent d'excellentes propriétés d'usage en application, notamment d'adhérence et de tenue sur l'ongle, ainsi qu'un excellent profil en terme de sécurité et de respect de la réglementation.

A la suite d'importantes recherches menées sur cette question, il a maintenant été trouvé que cet objectif, et d'autres, pouvaient être atteints au moyen d'une formulation particulière contenant une association unique d'ingrédients, qui permette de répondre de façon acceptable à l'ensemble des propriétés requises pour un vernis à ongles destiné à un usage commercial, tout en s'affranchissant de l'emploi de nitrocellulose.

### RESUME DE L'INVENTION

L'invention a ainsi pour premier objet une composition pour vernis à ongles, exempte ou ayant une concentration en nitrocellulose inférieure à ou, n'excédant pas 5000 ppm de nitrocellulose, et comprenant, dans un solvant organique cosmétiquement acceptable :
- un agent filmogène principal constitué de polyvinyl butyral,
- une silice pyrogénée hydrophobe,
- au moins une résine secondaire,
- au moins un plastifiant,
- au moins un colorant, insoluble dans ledit solvant.

Selon un mode de réalisation, la composition est exempte de nitrocellulose.

Selon un autre mode de réalisation, les colorants sont des pigments organiques ou minéraux.

Selon un autre mode de réalisation, la composition selon l'invention est non aqueuse.

Selon un mode particulièrement préféré de réalisation, la composition pour vernis à ongles selon l'invention est anhydre, exempte de nitrocellulose, et comprend, ou de préférence consiste en :
- un solvant organique cosmétiquement acceptable à hauteur de 10% à 95%, de préférence de 30% à 90%, et plus préférentiellement encore de 50% à 85%,
- un agent filmogène principal constitué de polyvinyl butyral à hauteur de 5% à 30%, de préférence de 5% à 25%, encore plus préférentiellement de 10% à 20%,
- une silice pyrogénée hydrophobe à hauteur de 0.1% à 15 %, de préférence de 0.3% à 10%, plus préférentiellement encore de 0.5% à 5%,
- au moins une résine secondaire à hauteur de 1% à 20%, de préférence de 3% à 20%, plus préférentiellement encore de 5% à 15%,
- au moins un plastifiant à hauteur de 0.1% à 10 %, de préférence de 0.5% à 8%, plus préférentiellement encore de 0.5% à 6%,
- au moins un pigment à hauteur de 0.001 % à 15%, de préférence de 0.005% à 12%, plus préférentiellement encore de 0.01 % à 10%,
les pourcentages ci-dessus étant exprimés en poids par rapport à l'ensemble de la composition.

Les avantages de l'invention sont multiples : les vernis à ongles peuvent être totalement exempts de nitrocellulose ; ils sont parfaitement stables chimiquement (absence de dégradation et/ou de changement de couleur) et physiquement (absence de sédimentation) ; ils sont d'une application extrêmement aisée ; et les films obtenus sur l'ongle après séchage présentent d'excellentes qualités.

Un second objet porte sur l'utilisation des compositions selon l'invention pour la protection et/ou le maquillage des ongles.

Un troisième objet concerne un procédé de protection et/ou de maquillage des ongles, qui consiste essentiellement à appliquer sur ces derniers une composition selon l'invention.

Un dernier objet, enfin, concerne des articles conditionnés, prêts à l'emploi, contenant la composition selon l'invention.

### DESCRIPTION DE MODES DE REALISATION DE L'INVENTION

L'invention va maintenant être décrite plus en détail et de façon non limitative dans les divers aspects qui la composent.

### Solvant organique

La composition pour vernis à ongles selon l'invention comprend au moins un solvant organique, permettant de solubiliser les substances polymériques qu'elle contient. Ce solvant peut ainsi être choisi parmi :
- les cétones liquides à température ambiante tels que la méthyléthyl cétone, l'acétone, la méthylisobutyl cétone ;
- les alcools liquides à température ambiante tels que l'éthanol, le propanol, le butanol, l'isopropanol, le diacétone d'alcool ;
- les esters à chaîne courte ayant de 3 à 8 atomes de carbone tels que l'acétate d'éthyle, l'acétate de propyle, l'acétate de butyle ;
- les alcanes liquides à température ambiante tels que l'heptane, le dodécane, l'hexane ;
- et leurs mélanges.

De préférence, le milieu solvant est anhydre ou substantiellement anhydre, c'est-à-dire que la composition se présente au final sous une forme non aqueuse, ou substantiellement non aqueuse, c'est-à-dire avec une teneur en poids d'eau inférieure à 1%.

Le solvant organique ou mélange de solvants est de préférence présent dans la composition dans une quantité comprise entre 10% et 95% en poids, de préférence entre 30% et 90% en poids, et plus préférentiellement encore entre 50% et 85% en poids, par rapport au poids total de la composition.

### Agent filmogène principal

La composition pour vernis à ongles selon l'invention comprend un agent filmogène principal qui est constitué de polyvinyl butyral.

Le polyvinyl butyral, appelé également poly(butyral vinylique) est un polymère thermoplastique de synthèse utilisé entre autres pour assembler les verres et fabriquer du vitrage feuilleté. Le polyvvinyl butyral peut être préparé à partir de l'acétate de vinyle qui par polymérisation radicalaire devient de l'acétate de polyvinyle. Ensuite l'acétate de polyvinyle est hydrolysé pour former l'alcool polyvinylique en présence d'un catalyseur acide. L'alcool polyvinylique subit ensuite une réaction d'acétalisation en présence du butanal (butyraldéhyde) en présence d'acide pour former le polyvinyl butyral.

Le polyvinyl butyral est un produit disponible dans le commerce, vendu notamment par la Société Kuraray sous les dénominations Mowital^{®}.

Selon un mode de réalisation, la composition pour vernis à ongles selon l'invention peut comprendre, à côté de l'agent filmogène principal, un ou plusieurs polymères filmogènes complémentaires connus en soi, comme par exemple une résine polyester ou une résine époxy tosylamide.

Selon un autre mode de réalisation, la composition ne contient aucun agent filmogène autre que le polymère polyvinyl butyral.

Le polymère filmogène principal constitué de polyvinyl butyral présente avantageusement une masse moléculaire moyenne comprise entre 10 000 et 100 000 g/mol et de préférence entre 15 000 et 60 000 g/mol.

Selon un mode préféré, le polymère filmogène principal constitué de polyvinyl butyral a une température de transition vitreuse Tg allant de 40°C à 100°C, de préférence de 60°C à 80°C.

La quantité d'agent filmogène principal constitué de polyvinyl butyral dans la composition pour vernis à ongles selon l'invention peut être de 5% à 30% en poids, de préférence de 5% à 25%, plus préférentiellement de 10% à 20%, par rapport au poids total de la composition.

Selon l'invention, la composition est exempte, ou présente une concentration en nitrocellulose inférieure à, ou n'excédant pas, 5 000 ppm, de préférence inférieure à 1 000 ppm, plus préférentiellement encore inférieure à 500 ppm, et encore plus préférentiellement inférieure à 100 ppm.

### Résine secondaire

La composition pour vernis à ongles selon l'invention comprend en outre au moins une résine secondaire.

Cette résine secondaire, qui peut avoir ou non des propriétés filmogènes, peut être choisie parmi les résines acryliques, les résines styrène acryliques, les résines polyesters, les résines alkydes, les résines polyuréthanes, les résines cétoniques, les résines époxy tosylamide, les résines époxy, les résines polyamides, les résines polyacétate de vinyle, et leurs mélanges. L'utilisation de ces résines est bien connue dans le domaine de la cosmétique, en particulier celui des vernis à ongles.

A titre d'exemple, on peut citer les résines commercialement disponibles suivantes : Elvacite^{®} 2046 de la société Lucite (résine acrylique), Beckosol^{®} OD 230-70-E de la société Dainippon (résine alkyde), Trixene^{®} PR 4127 de la société Baxenden (résine polyuréthane), Variplus^{®} SK de la société Tego (résine cétonique) et Polytex^{®} NX 55 de la Société Estron Chemical (résine époxy tosylamide).

Par résine secondaire, on entend un ou plusieurs polymère(s) en moindre proportion pondérale que l'agent filmogène principal constitué de polyvinyl butyral.

Dans une formulation de vernis à ongle, la fonction principale d'une résine secondaire est de permettre d'augmenter la brillance et l'adhésion sur l'ongle.

La quantité de résine(s) secondaire(s) dans la composition pour vernis à ongles selon l'invention peut aller de 1% à 20% en poids, de préférence de 3% à 20%, plus préférentiellement encore de 5% à 15%, par rapport au poids total de la composition.

### Plastifiant

La composition pour vernis à ongles selon l'invention comprend également au moins un plastifiant.

De façon connue dans le domaine, la fonction du plastifiant est de permettre d'ajuster le compromis dureté/flexibilité du film.

Selon l'invention le plastifiant peut ainsi être choisi, seul ou en mélange, parmi :
- les esters d'acides, notamment carboxyliques, tels que les citrates, les benzoates, les adipates et les carbonates ;
- les diesters d'isosorbide.

L'isosorbide est un produit obtenu par déshydratation d'un dérivé du glucose, le sorbitol, qui peut être extrait de baies de sorbier ou de céréales. Le diester est avantageusement produit par réaction entre un acide gras d'origine végétale et l'isosorbide.

Parmi les ester de citrates, on peut citer à titre d'exemple, le triéthyl citrate, le tributyl citrate, le tributyl acétylcitrate. Et, parmi les esters de benzoates, on peut citer à titre d'exemple, le triméthyl pentanediol dibenzoate.

Le plastifiant est avantageusement présent dans la composition pour vernis à ongles dans une concentration comprise entre 0.1% et 10 % en poids, de préférence entre 0.5% et 8%, plus préférentiellement encore entre 0.5% et 6%, en poids par rapport au poids total de la composition.

### Silice pyrogénée

La composition pour vernis à ongles selon l'invention comprend de la silice pyrogénée hydrophobe.

Les silices pyrogénées hydrophobes peuvent être obtenues par modification de la surface de la silice par une réaction chimique générant une diminution du nombre de groupes silanols, ces groupes pouvant être notamment substitués par des groupements hydrophobes. Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous la référence "AEROSIL^{®} R812" par la Société Evonik.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous la référence "AEROSIL^{®} R972" par la Société Evonik.

Les silices pyrogénées hydrophobes présentent de façon générale des surfaces spécifiques (mesurée selon la méthode BET) élevées, en particulier supérieures à 30 m²/g, et encore plus particulièrement supérieures à 100 m²/g.

Selon l'invention, on préfère utiliser des silices pyrogénées dont la surface spécifique est comprise entre 50 m²/g et 380 m²/g, et encore plus avantageusement entre 200 m²/g et 300 m²/g.

La silice pyrogénée hydrophobe peut être présente dans la composition pour vernis à ongles en une teneur comprise entre 0.1% et 15 % en poids, de préférence entre 0.3% et 10%, plus préférentiellement encore entre 0.5% et 5%, en poids par rapport au poids total de la composition.

### Colorant

La composition pour vernis à ongles selon l'invention comprend en outre au moins un colorant choisi par exemple parmi les pigments, les nacres et les paillettes.

Par pigments, il faut comprendre des particules de toute forme, blanches ou colorées, minérales ou organiques, naturelles ou synthétiques, insolubles dans le milieu de formulation (solvant), et destinées à apporter de la couleur à la composition.

Par nacres, il faut comprendre des particules de toute forme irisées, notamment produites par certains mollusques dans leur coquille ou bien synthétisées. Les principaux substrats des nacres sont les suivants : Mica naturel, fluorophlogopite, borosilicate, aluminium, et autres. Ces substrats sont ensuite recouverts soit uniquement de dioxyde de titane, ce qui correspond aux nacres blanches iridescentes, soit de différentes couches de pigments minéraux (dioxyde de titane, oxyde de fer, ferrocyanure de fer, et autres) et de pigment organiques (tels que red 7, red 34, yellow 5, et autres).

Concernant les paillettes, les substrats principaux sont le polyéthylène téréphtalate et le poly butylène téréphtalate. Les résines utilisées pour recouvrir la fine couche d'aluminium et/ou colorer la paillette sont à base de polyuréthane 11, polyuréthane 33, copolymère acryliques ou copolymère acétate de vinyle/éthylène.

Les pigments peuvent être blancs ou colorés, minéraux et/ou organiques. On peut ainsi citer, parmi les pigments minéraux le dioxyde de titane, les oxydes de zirconium ou de cérium, les oxydes de zinc, les oxydes de chrome, les différentes nuances d'oxyde de fer (noir, jaune ou rouge), le bleu d'outremer, le ferrocyanure de fer, le violet de manganèse, le bleu outremer et autres.

Parmi les pigments organiques on peut citer plus particulièrement le Red 7, le Red 34, le Yellow 5, et plus généralement les pigments insolubles de type D&C.

Les pigments nacrés peuvent se présenter sous la forme par exemple de particules de mica ou de borosilicate revêtues d'une ou plusieurs couches d'oxyde de titane et/ou d'oxyde de fer, permettant de conférer, par réflexion et réfraction de la lumière, des reflets au vernis à l'état sec.

Le pigment, ou le mélange de pigments, est de préférence présent dans la composition dans une quantité comprise entre 0.001% et 15% en poids, de préférence entre 0.005% et 12% en poids, plus préférentiellement encore entre 0.01% et 10% en poids, par rapport au poids total de la composition.

Les compositions selon l'invention peuvent éventuellement contenir, en plus des colorants insolubles précités, un ou plusieurs colorants qui seraient solubles, au moins pour partie, dans le solvant cosmétiquement acceptable mis en oeuvre.

### Additifs éventuels

A côté des ingrédients essentiels précédemment définis, la composition selon l'invention peut en outre, de façon facultative, contenir des additifs complémentaires usuels dans le domaine de la cosmétique, comme par exemple des agents anti-UV, des agents anti-oxydants, des agents de surface comme des silicones, des parfums, ou encore des actifs tels que des vitamines, des protéines ou des extraits végétaux.

### Préparation des compositions selon l'invention

Les compositions selon l'invention peuvent être préparées selon des méthodes classiques bien connues de l'homme de l'art dans le domaine de la formulation des vernis à ongles. Par ailleurs, des méthodes de préparation de ces compositions sont illustrées dans les exemples donnés ci-après.

### Articles conditionnés et utilisation des compositions selon l'invention

Les compositions selon l'invention peuvent être conditionnées dans tout type de récipients ou flacons connus en soi, en verre ou en plastique par exemple, équipés à l'une de leurs extrémités d'un système d'ouverture et de fermeture amovible (tel qu'un bouchon vissable/dévissable) permettant d'en assurer l'étanchéité aux fins d'une bonne conservation.

Les bouchons peuvent être munis d'un applicateur (pinceau, spatule, embout) qui, après trempage dans la composition, permet l'application sur l'ongle de cette composition. Selon une autre variante, l'applicateur n'est pas solidarisé au bouchon et constitue un article séparé pour former un kit en deux parties de type récipient/applicateur.

Avant emploi, la viscosité de la composition conditionnée (au repos) est généralement comprise entre 1 500 et 3 000 cP (selon mesure Brookfield précisée dans les exemples).

Au moment de l'emploi, après agitation mécanique et/ou trempage de l'applicateur, la viscosité de la composition baisse pour être alors généralement comprise entre 400 et 1 500 cP (selon mesure Brookfield précisée dans les exemples), de façon à permettre sa parfaite application sur l'ongle par l'utilisatrice.

Après application sur l'ongle, la composition retrouve progressivement sa viscosité initiale et sèche (à température ambiante et/ou par apport de chaleur pour accélérer le séchage), de façon à former au final un film de revêtement continu et durable sur l'ongle.

Si l'utilisatrice le désire, il est ensuite possible d'éliminer le film de vernis à ongle au moyen d'un dissolvant classique, comme par exemple de l'acétate d'éthyle.

### EXEMPLES

On a préparé des produits conformes à l'invention et des produits comparatifs. Les matières premières utilisées pour la préparation de ces produits étaient les suivantes :
- **Mowital^{®}** : polyvinyl butyral (agent filmogène principal) commercialisé par la Société Kuraray
- **CAB 381** - **0,5^{®}** : ester acétate/butyrate de cellulose (agent filmogène principal) commercialisé par la Société Eastman
- **Polytex E75^{®}** : résine secondaire de type époxy tosylamide commercialisée par la Société Estron Chemical
- **Aerosil R812^{®}** : silice pyrogénée hydrophobe commercialisée par la Société Evonik
- **Sorbosil BFG50^{®}** : silice hydratée commercialisée par la Société PQ Corporation
- **Tixogel MP Z^{®}** : bentonite hydrophobe commercialisée par la Société BYK
- **Uvasorb 20H^{®}** : filtre UV organique (benzophénone-1) commercialisé par la Société 3V

Sur les produits préparés, on a réalisé les tests suivants:
Brillance : sur une plaque de type Leneta, on applique une couche de 100 µm d'épaisseur de la composition de vernis. Après séchage à 20 °C du film obtenu, on mesure sa brillance (Gloss, UB) sous un angle d'incidence de 60° à l'aide d'un glossmètre Byk Gardner.
Dureté : la dureté «Persoz» est mesurée sur une plaque de verre recouverte d'un vernis de 100 µm humide (selon la norme ISO1522). Une valeur minimale de 200 est souhaitée, préférentiellement supérieure à 210.
Adhérence : le «Cross hatch test» est réalisé sur une plaque de verre. Un film de 100µm humide est appliqué et séché 24h à 20°C. Un quadrillage est réalisé au moyen d'un couteau à plusieurs lames. Un ruban adhésif est collé sur ce quadrillage, puis retiré : on analyse alors le quadrillage. La note 0 correspond à l'absence de perte d'adhésion (aucun carré n'a été enlevé). La note 5 correspond à la perte totale d'adhésion (tous les carrés ont été enlevés). Une note comprise entre 0 et 1 est indispensable.
Stabilité : Une teinte contenant un ou des pigments organiques est réalisée. Un film de 400µm d'épaisseur est réalisé sur une carte et séché. Ce film est analysé grâce à un spectro photo colorimètre. Après 24h à 20°C, un nouveau film est appliqué et analysé de la même manière que précédemment. Le comparatif des valeurs de **L**, **a**, **b** nous donne une information sur l'évolution de la couleur dans le temps.
Viscosité : elle est mesurée à l'aide d'un viscosimètre BROOKFIELD DVIII+ en utilisant l'aiguille N°3. L'échantillon à mesurer est placé dans un bain thermostaté à 25°C durant 12 heures. L'aiguille est ensuite introduite dans cet échantillon et la mesure de la viscosité est réalisée à une vitesse de 60rpm.

Première étape de fabrication : fabrication de produits sous forme de gel (produits sans plastifiant et sans pigment)

### (i) Compositions :

| **Exemple N°** | **1** | **2** | **3** | **4** | **4A** |
|---|---|---|---|---|---|
| Acétate de butyle | 74 | 73 | 74 | 73 | 74 |
| Polytex E75 | 10 | 10 | 10 | 10 | 10 |
| Aerosil R812 | 7 | 0 | 7 | 0 | 0 |
| Tixogel MP Z | 0 | 6 | 0 | 6 | 0 |
| Sorbosil BFG50 | 0 | 0 | 0 | 0 | 7 |
| Mowital 30HH | 9 | 9 | 0 | 0 | 9 |
| CAB 381-0.5 | 0 | 0 | 9 | 9 | 0 |
| Diacétone alcool | 0 | 1 | 0 | 1 | 0 |

Le produit de l'exemple 1 présente un schéma de formulation conforme à l'invention (polyvinyl butyral + silice pyrogénée), les autres exemples étant des comparatifs.

### (ii) Procédé de fabrication :

Dans un bécher de 2L, on charge l'acétate de butyle et soit l'Aerosil R812 soit le Tixogel MP Z soit le Sorbosil BFG50. On agite pour le désagglomérer. On incorpore ensuite le Polytex E75 sous agitation. On agite 20 minutes. On introduit la Diacétone alcool si nécessaire. On introduit ensuite le Mowital 30HH ou le CAB 381-0.5 en fonction de la formule et on agite 10 minutes. Le mélange obtenu est ensuite broyé dans un broyeur à bille. Un gel est alors obtenu.

On a mesuré sur les produits ainsi préparés leur viscosité et leur brillance :

| **Exemple N°** | **1** | **2** | **3** | **4** | **4A** |
|---|---|---|---|---|---|
| Viscosité (60rpm, 25°C) | 2500 | 9200 | 9200 | 8700 | 200 |
| Brillance (60°) | 48,5 | 58,1 | 24,6 | 64,1 | 32 |

Pour le produit 4A, on observe une sédimentation de la silice.

Seconde étape de fabrication : fabrication des bases vernis (sans pigment)

### (i) Compositions :

| **Exemple N°** | **5** | **6** | **7** | **8** | **8A** |
|---|---|---|---|---|---|
| Acétate de butyle | 22.8 | 22.77 | 22.8 | 22.77 | 22.77 |
| Acétate d'éthyle | 35 | 35 | 35 | 35 | 35 |
| Mowital 30HH | 9 | 9 | 0 | 0 | 9 |
| Mowital 60HH | 3 | 3 | 0 | 0 | 3 |
| CAB 381-05 | 0 | 0 | 12 | 12 | 0 |
| Uvasorb 20H | 0.2 | 0.2 | 0.2 | 0.2 | 0.2 |
| Acétyl tributyl citrate | 1 | 1 | 3 | 3 | 1 |
| Polytex E75 | 9 | 9 | 9 | 9 | 9 |
| Gel Exemple 1 | 20 | 0 | 0 | 0 | 0 |
| Gel Exemple 2 | 0 | 20 | 0 | 0 | 0 |
| Gel Exemple 3 | 0 | 0 | 20 | 0 | 0 |
| Gel Exemple 4 | 0 | 0 | 0 | 20 | 0 |
| Gel Exemple 4A | 0 | 0 | 0 | 0 | 20 |
| Acide phosphorique | 0 | 0.03 | 0 | 0.03 | 0 |

Le produit de l'exemple 5 présente un schéma de formulation conforme à l'invention (polyvinyl butyral + silice pyrogénée), les autres exemples étant des comparatifs.

### (ii) Procédé de fabrication :

Dans un bécher de 2L, on charge l'acétate de butyle et l'acétate d'éthyle. Sous agitation, on introduit le Mowital 30HH, puis le Mowital 60HH ou le CAB 381-0.5 en fonction de la formule. Une fois solubilisé, on introduit l'Uvasorb 20H, le Polytex E75, l'Acetyl Tributyl Citrate et, selon la formule, le gel des exemples 1 à 4A précédents. On agite 15 minutes. Le cas échéant, l'acide phosphorique est ajouté et le mélange est agité 15 minutes de plus. Une base vernis est obtenue avec les caractéristiques suivantes :

| **Exemple N°** | **5** | **6** | **7** | **8** | **8A** |
|---|---|---|---|---|---|
| Viscosité Brookfield (60rpm, 25°C, mPa.s) | 700 | 690 | 900 | 760 | 400 |
| Brillance (60°) | 89 | 90,2 | 70,8 | 87,4 | 50 |
| Dureté | 289 | 282 | 240 | 225 | - |
| Adhésion | 0 | 0 | 5 | 0 | 5 |

### Fabrication des produits finis et évaluation de la stabilité de la couleur et de la tenue sur l'ongle

Pour réaliser des teintes, il a fallu d'abord broyer des pigments. Les compositions suivantes ont été réalisées :

| **Exemple N°** | **9** | **10** | **11** | **12** |
|---|---|---|---|---|
| Acétate de butyle | 17 | 17 | 27 | 27 |
| Acétate d'éthyle | 19 | 19 | 29 | 29 |
| Isopropanol | 3 | 3 | 5 | 5 |
| Polytex E75 | 6 | 6 | 10 | 10 |
| Mowital 30HH | 7 | 0 | 11 | 0 |
| CAB 381-0.5 | 0 | 7 | 0 | 11 |
| Acétyl tributyl citrate | 8 | 8 | 8 | 8 |
| Dioxyde de titane | 40 | 40 | 0 | 0 |
| Red 34 | 0 | 0 | 10 | 10 |

Dans un bécher de 2L, on charge l'acétate de butyle, l'acétate d'éthyle et l'isopropanol. On incorpore ensuite le Polytex E75 et le Mowital 30HH ou le CAB 381-0.5 en fonction de la formule, sous agitation. Une fois le Mowital dissout, on ajoute le pigment et on agite 30 minutes. Le mélange obtenu est ensuite broyé dans un broyeur à bille. Une solution colorante est alors obtenue.

La teinte suivante a été ensuite réalisée sous la forme d'un produit fini pour démontrer l'amélioration de la stabilité de la couleur.

| **Exemple N°** | **13** | **14** | **15** | **16** | **16A** |
|---|---|---|---|---|---|
| Composition Exemple 5 | 97.9 | 0 | 0 | 0 | 0 |
| Composition Exemple 6 | 0 | 97.9 | 0 | 0 | 0 |
| Composition Exemple 7 | 0 | 0 | 97.9 | 0 | 0 |
| Composition Exemple 8 | 0 | 0 | 0 | 97.9 | 0 |
| Composition Exemple 8A | 0 | 0 | 0 | 0 | 97.9 |
| Composition Exemple 9 | 2 | 2 | 0 | 0 | 2 |
| Composition Exemple 10 | 0 | 0 | 2 | 2 | 0 |
| Composition Exemple 11 | 0.1 | 0.1 | 0 | 0 | 0.1 |
| Composition Exemple 12 | 0 | 0 | 0.1 | 0.1 | 0 |

Seul le produit de l'exemple 13 correspondant à un produit conforme à l'invention, les autres exemples correspondant à des produits comparatifs (du fait que la silice pyrogénée a été substituée par de la bentonite et/ou par de la silice hydratée et/ou du fait que le polyvinyl butyral a été substitué par un ester de cellulose). Tous les produits présentent le même système colorant, à savoir un mélange TiO2 + Red 34, à la même concentration.

Une mesure de couleur (système L, a, b) a été réalisée à T0 et T + 24h sur les produits ainsi préparés. Les résultats ci-dessous ont été obtenus :

| **Exemple N°** | **13** | **14** | **15** | **16** | **16A** |
|---|---|---|---|---|---|
| **L** | 76.46 | 76.54 | 75.14 | 75.01 | 76.85 |
| **L** + 24h | 76.98 | 76.10 | 75.39 | 74.8 | 75.16 |
| **a** | 22.61 | 23.53 | 24.26 | 24.04 | 21.41 |
| **a** + 24h | 22.04 | 30.48 | 24.21 | 35.28 | 23.54 |
| **b** | -8.99 | -8.56 | -10.02 | -10.28 | -10.21 |
| **b** + 24h | -8.63 | -5.09 | -9.79 | -8.04 | -10.56 |

On observe une très faible différence au niveau de la variation des 3 composantes de la couleur entre la mesure à T0 et la mesure après 24h pour les exemples 13 et 15. Pour les exemples 14 et 16, cette variation est très forte. On passe ainsi d'un rose bleuté à un rose jaune. Pour l'exemple 16A, on observe une diminution du **L** (qui correspond à une perte de blanc) et une augmentation du **a** (qui correspond à une augmentation du rouge). Ceci est dû à l'incapacité de la silice hydratée à stabiliser les pigments : on observe une sédimentation du dioxyde de titane et donc une teinte moins blanche et plus rouge.

On a ensuite évalué la tenue des vernis sur l'ongle.

Pour cela, un test in vivo a été réalisé sur un panel de 20 personnes. Ces panelistes ont donné leur avis sur la tenue du vernis appliqué sur l'ongle, en indiquant le nombre de jours où cette tenue a été observée. La moyenne des réponses est présentée dans le tableau ci-dessous :

| **Exemple N°** | **13** | **14** | **15** | **16** |
|---|---|---|---|---|
| Nombre de jour noté de tenue sur l'ongle (moyenne) | 4.8 | 4.3 | 1.4 | 1.8 |

On observe une très mauvaise tenue sur l'ongle des vernis 15 et 16 qui ne dépassent en effet pas 2 jours. A l'inverse, les vernis 13 et 14 ont une très bonne tenue proche de 5 jours.

Pour terminer, on a procédé sur les compositions des exemples 13 à 16 aux mesures suivantes :

| **Exemple N°** | **13** | **14** | **15** | **16** |
|---|---|---|---|---|
| Viscosité Brookfield (60rpm, 25°C, mPa.s) | 900 | 780 | 960 | 620 |
| Brillance (60°) | 86.9 | 86.4 | 52.9 | 78.3 |
| Dureté | 267 | 263 | 225 | 204 |
| Adhésion | 0 | 0 | 5 | 0 |

En conclusion, on constate donc que le vernis à ongle de l'exemple 13 qui est conforme à l'invention est celui qui présente les meilleures propriétés à la fois en termes de stabilité de couleur et de tenue sur l'ongle. Par ailleurs, ce vernis à ongles présente également de très bonnes propriétés de brillance, de dureté et d'adhésion (comme illustré également par l'exemple 5).

## Revendications

1. Composition pour vernis à ongles exempte ou ayant une concentration en nitrocellulose inférieure à ou, n'excédant pas 5000 ppm de nitrocellulose comprenant, dans un solvant organique cosmétiquement acceptable :
- un agent filmogène principal constitué de polyvinyl butyral,
- une silice pyrogénée hydrophobe,
- au moins une résine secondaire,
- au moins un plastifiant,
- au moins un colorant, insoluble dans ledit solvant.

2. Composition selon la revendication 1, **caractérisée par le fait qu'**elle présente une teneur en poids d'eau inférieur à 1%.

3. Composition selon l'une des revendications 1 à 2, **caractérisée par le fait que** le solvant organique représente de 10% à 95%, de préférence de 30% à 90%, et plus préférentiellement encore de 50% à 85%, du poids total de la composition.

4. Composition selon l'une des revendications 1 à 3, **caractérisée par le fait que** le polyvinyl butyral présente une masse moléculaire moyenne comprise entre 10 000 et 100 000 g/mol, de préférence entre 15 000 et 60 000 g/mol.

5. Composition selon l'une des revendications 1 à 4, **caractérisée par le fait que** le polyvinyl butyral a une température de transition vitreuse Tg allant de 40°C à 100°C, de préférence de 60°C à 80°C.

6. Composition selon l'une des revendications 1 à 5, **caractérisée par le fait que** le polyvinyl butyral représente de 5% à 30%, de préférence de 5% à 25%, encore plus préférentiellement de 10% à 20%, du poids total de la composition.

7. Composition selon l'une des revendications 1 à 6, **caractérisée par le fait que** la silice pyrogénée présente une surface spécifique comprise entre 50 m²/g et 380 m²/g, de préférence entre 200 m²/g et 300 m²/g.

8. Composition selon l'une des revendications 1 à 7, **caractérisée par le fait que** la silice pyrogénée hydrophobe représente de 0.1% à 15 % en poids, de préférence de 0.3% à 10%, plus préférentiellement encore de 0.5% à 5%, du poids total de la composition.

9. Composition selon l'une des revendications 1 à 8, **caractérisée par le fait que** le colorant est un pigment organique ou minéral.

10. Composition selon la revendication 9, **caractérisée par le fait que** le pigment représente de 0,001% à 15%, de préférence de 0,005% à 12%, plus préférentiellement encore de 0,01% à 10%, du poids total de la composition.

11. Composition selon l'une des revendications 1 à 10, **caractérisée par le fait que** la ou les résines secondaires représentent de 1% à 20%, de préférence de 3% à 20%, plus préférentiellement encore de 5% à 15%, du poids total de la composition.

12. Composition selon l'une des revendications 1 à 11, **caractérisée par le fait que** le ou les plastifiants représentent de 0,1% à 10 %, de préférence de 0,5% à 8%, plus préférentiellement encore de 0,5% à 6%, du poids total de la composition.

13. Utilisation d'une composition telle que définie à l'une quelconque des revendications 1 à 12 pour la protection et/ou le maquillage des ongles.

14. Procédé de protection et/ou de maquillage des ongles, qui comprend l'application sur lesdits ongles d'une composition telle que définie à l'une quelconque des revendications 1 à 12.

15. Article comprenant (i) un récipient muni d'un système de fermeture et rempli d'une composition telle que définie à l'une quelconque des revendications 1 à 12 et (ii) un applicateur, solidarisé ou non audit système de fermeture, et destiné, après trempage dans ledit récipient, à déposer ladite composition sur un ongle.

## Patentansprüche

1. Zusammensetzung für Nagellack, die frei von Nitrocellulose ist oder eine Konzentration an Nitrocellulose von weniger als oder nicht mehr als 5.000 ppm Nitrocellulose aufweist, die in einem kosmetisch akzeptablen organischen Lösungsmittel Folgendes umfasst:
- einen Hauptfilmbildner, der aus Polyvinylbutyral besteht,
- ein hydrophobes pyrogenes Siliciumdioxid,
- mindestens ein sekundäres Harz,
- mindestens einen Weichmacher,
- mindestens einen Farbstoff, der in dem Lösungsmittel unlöslich ist.

2. Zusammensetzung nach Anspruch 1, **dadurch gekennzeichnet, dass** sie einen Wassergehalt von weniger als 1 Gew.-% aufweist.

3. Zusammensetzung nach einem der Ansprüche 1 bis 2, **dadurch gekennzeichnet, dass** das organische Lösungsmittel 10 % bis 95 %, vorzugsweise 30 % bis 90 % und noch bevorzugter 50 % bis 85 %, des Gesamtgewichts der Zusammensetzung ausmacht.

4. Zusammensetzung nach einem der Ansprüche 1 bis 3, **dadurch gekennzeichnet, dass** das Polyvinylbutyral ein mittleres Molekulargewicht zwischen 10.000 und 100.000 g/mol, vorzugsweise zwischen 15.000 und 60.000 g/mol, aufweist.

5. Zusammensetzung nach einem der Ansprüche 1 bis 4, **dadurch gekennzeichnet, dass** das Polyvinylbutyral eine Glasübergangstemperatur Tg von 40 °C bis 100 °C, vorzugsweise von 60 °C bis 80 °C, aufweist.

6. Zusammensetzung nach einem der Ansprüche 1 bis 5, **dadurch gekennzeichnet, dass** das Polyvinylbutyral 5 % bis 30 %, vorzugsweise 5 % bis 25 %, noch bevorzugter 10 % bis 20 %, des Gesamtgewichts der Zusammensetzung ausmacht.

7. Zusammensetzung nach einem der Ansprüche 1 bis 6, **dadurch gekennzeichnet, dass** das pyrogene Siliciumdioxid eine spezifische Oberfläche zwischen 50 m²/g und 380 m²/g, vorzugsweise zwischen 200 m²/g und 300 m²/g aufweist.

8. Zusammensetzung nach einem der Ansprüche 1 bis 7, **dadurch gekennzeichnet, dass** das hydrophobe pyrogene Siliciumdioxid 0,1 % bis 15 % Gew.-%, vorzugsweise 0,3 Gew.-% bis 10 Gew.-%, besonders bevorzugt 0,5 Gew.-% bis 5 Gew.-%, des Gesamtgewichts der Zusammensetzung ausmacht.

9. Zusammensetzung nach einem der Ansprüche 1 bis 8, **dadurch gekennzeichnet**, der Farbstoff ein organisches oder anorganisches Pigment ist.

10. Zusammensetzung nach Anspruch 9, **dadurch gekennzeichnet, dass** das Pigment 0,001 % bis 15 %, vorzugsweise 0,005 % bis 12 %, noch bevorzugter 0,01 % bis 10 %, des Gesamtgewichts der Zusammensetzung ausmacht.

11. Zusammensetzung nach einem der Ansprüche 1 bis 10, **dadurch gekennzeichnet, dass** das sekundäre Harz oder die sekundären Harze 1 % bis 20 %, vorzugsweise 3 % bis 20 %, noch bevorzugter 5 % bis 15 %, des Gesamtgewichts der Zusammensetzung ausmachen.

12. Zusammensetzung nach einem der Ansprüche 1 bis 11, **dadurch gekennzeichnet, dass** der oder die Weichmacher 0,1 % bis 10 %, vorzugsweise 0,5 % bis 8 %, noch bevorzugter 0,5 % bis 6 %, des Gesamtgewichts der Zusammensetzung ausmachen.

13. Verwendung einer Zusammensetzung, wie in einem der Ansprüche 1 bis 12 definiert, zum Schützen und/oder zum Verschönern der Nägel.

14. Verfahren zum Schützen und/oder Verschönern von Nägeln, das das Auftragen einer Zusammensetzung, wie sie in einem der Ansprüche 1 bis 12 definiert ist, auf die Nägel umfasst.

15. Gegenstand, umfassend (i) einen Behälter, der mit einem Verschlusssystem versehen und mit einer Zusammensetzung gefüllt ist, wie sie in einem der Ansprüche 1 bis 12 definiert ist, und (ii) einen Applikator, der mit dem Verschlusssystem fest verbunden ist oder nicht und dazu bestimmt ist, nach dem Eintauchen in den Behälter die Zusammensetzung auf einen Nagel aufzutragen.

## Claims

1. A composition for nail varnish, which is free of nitrocellulose or having a nitrocellulose concentration of less than, or not exceeding, 5,000 ppm, and which includes, in a cosmetically acceptable organic solvent:
- a primary film-forming agent consisting of polyvinyl butyral,
- a fumed silica,
- at least one secondary resin,
- at least one plasticiser,
- at least one colourant, which is insoluble in said solvent.

2. The composition of claim 1, **characterized by** the fact that it has a water content by weight of less than 1%.

3. The composition of one of claims 1 to 2, **characterized by** the fact that the organic solvent represents from 10% to 95%, preferably from 30% to 90%, and more preferentially from 50% to 85%, of the total weight of the composition.

4. The composition of one of claims 1 to 3, **characterized by** the fact that the polyvinyl butyral has an average molecular mass comprised between 10,000 and 100,000 g/mol, preferably between 15,000 and 60,000 g/mol.

5. The composition of one of claims 1 to 4, **characterized by** the fact that the polyvinyl butyral has a glass-transition temperature Tg ranging from 40°C to 100°C, preferably from 60°C to 80°C.

6. The composition of one of claims 1 to 5, **characterized by** the fact that the polyvinyl butyral represents from 5% to 30%, preferably from 5% to 25%, more preferentially from 10% to 20%, of the total weight of the composition.

7. The composition of one of claims 1 to 6, **characterized by** the fact that the fumed silica has a specific surface area comprised between 50 m²/g and 380 m²/g, preferably between 200 m²/g and 300 m²/g.

8. The composition of one of claims 1 to 7, **characterized by** the fact that the fumed silica represents from 0.1% to 15% by weight, preferably from 0.3% to 10%, more preferentially from 0.5% to 5%, of the total weight of the composition.

9. The composition of one of claims 1 to 8, **characterized by** the fact that the colourant is an organic or mineral pigment.

10. The composition of claim 9, **characterized by** the fact that the pigment represents from 0.001% to 15%, preferably from 0.005% to 12%, more preferentially from 0.01% to 10%, of the total weight of the composition.

11. The composition of one of claims 1 to 10, **characterized by** the fact that the one or more secondary resins represent from 1% to 20%, preferably from 3% to 20%, more preferentially from 5% to 15%, of the total weight of the composition.

12. The composition of one of claims 1 to 11, **characterized by** the fact that the one or more plasticisers represent from 0.1% to 10%, preferably from 0.5% to 8%, even more preferentially from 0.5% to 6%, of the total weight of the composition.

13. Use of a composition as defined in any one of claims 1 to 12 for protecting and/or making-up the nails.

14. A method for protecting and/or making-up the nails, which comprises applying a composition as defined in any one of claims 1 to 12 on said nails.

15. An item comprising (i) a container provided with a closure system and filled with a composition as defined in any one of claims 1 to 12 and (ii) an applicator, optionally integral with said closure system, and intended, after dipping in said container, to deposit said composition on a nail.
